# EUROPEAN PATENT APPLICATION

(11) **EP 1 202 064 A2**
(43) Date of publication of application: **02.05.2002**
(21) Application number: 01118980.0
(22) Date of filing: 06.08.2001
(51) Int. Cl.: G01N 33/569, C12N 1/14, A61K 35/70, A61K 35/72

(54) **Method for detecting antibodies to and antigens of fungal and yeast exposures**

(30) Priority: 27.10.2000 CA 2325063
(71) Applicant: The Minister of National Defence Government of Canada, Ottawa, Ontario K1A OK2 (CA)
(72) Inventor: Cherwonogrodzky, John W., Alberta, T1B 3L5 (CA)
(74) Representative: Goddar, Heinz J., Dr.

(57) **Abstract**

A serodiagnostic assay for fungal and yeast antibodies in animal and human test subjects is disclosed. The assay is comprised of preparing fungal and yeast cell culture supernatants mixture, reacting the cell culture supernatants with sera from a test subject, and determining the serum antibody level of the test subject. The present invention also discloses some unique characteristics of yeast and fungal supernatants which enhance the accuracy and efficacy of the serodiagnostic assay of the invention.

## Description

### Field of the Invention

This invention relates to a serodiagnostic assay for detecting fungal or yeast infection by screening fungal or yeast antibodies using cell culture supernatant as the source of fungal or yeast antigens.

### Background of the Invention

Diseases resulted from fungal or yeast infections are becoming a serious public health concern. In order to combat this rising threat, the medical and research communities have been endeavouring to develop diagnostic tools for detecting the presence of fungal or yeast antibodies in a more reliable, accurate and cost effective manner.

Until now, laboratory assays to determine fungal infections were cumbersome and insensitive. For example, Balows et al. (1991), *American Society for Microbiology Press*, Chapters 57-65, pp. 579-700, teaches methods which included histological microscopic examinations of tissue biopsies, isolation and culturing over lengthy periods such as a month.

Alternative methods were also not encouraging. In Patterson et al. (1996), *J. Clinical Microbiol.,* 34:1794-1797, level of serum *Aspergillus* carbohydrate antigen was determined by enzyme-linked immunosorbent assay (ELISA) and used as an indicator for *Aspergillosis*. However, the sensitivity and level of detection of the targeted antigen remained relatively low and, therefore, efficacy of the assay was greatly diminished (e.g. only 13% of patients with invasive pulmonary aspergillosis could be identified).

Similarly, assessing for fungal components by using rabbit antisera gave very low detection rate. Likewise, DNA detection through polymerase chain reaction (PCR) appeared to be overly specific, such that the PCR detects DNA from *Aspergillus fumigatus* but not from any other *Aspergillus* (see Spreadbury et al. (1993), *J. Clinical Microbiol.,* 31:615-621).

In U.S. Patent No. 5,932,705 (issued Aug. 3, 1999), Berget et al. teach the use of cell-free *Pasteurella haemolytica* supernatants as individual antigen compositions which are identified through reactions with sera from infected cattle for diagnosing and treating bovine pasteurellosis (Shipping Fever). However, unlike this pathogen, not all micro-organisms shed toxins or components into culture supernatants. Indeed, in the diagnostic laboratory, few are identified in this manner and most often organisms are isolated, cultured and identified using the whole cell. This is especially true for fungi or yeast where current serological methods are ineffective (see Balows et al., *supra).* Accordingly, culture supernatant proteins are believed to correspond to those proteins which are released from the bacteria during active *P. haemolytica* infections and are more likely to include leukotoxin components thought to be involved in the breakdown of immunosurveillance in infected cattle, or other components not present in bacterins. However, Berget et al. did not extend their work further to detecting yeast and fungal antigens using different cell culture supernatants.

Despite a lack of success for immunological methods as reported in the literature, the present invention provides a different means such that serum antibodies are detected in test subjects which are exposed to yeast or fungal antigens with high degree of specificity and reliability.

### Other Literatures of General Interest:

Taylor T. (1996), Iraq's biological weapons program. *Chemical and Biological Defense Information Analysis Center (CBIAC) Newsletter*, 2 (2) 3-4.
Zilinskas, R.A. (1997), Iraq's biological weapons, the past as future? *JAMA*, 278 (5) 418-424.
Campbell, C.K. (1995), Hazards to laboratory staff posed by fungal pathogens. *Journal of Hospital Infection (suppl.)*, 30: 358-363.
Fox, J.L. (1993), Fungal infection rates are increasing. *ASM News,* 59 (10) 515-518.
Homer, W.E., A. Helbling, J.E. Salvaggio and S.B. Lehrer. (1995), Fungal allergens. *Clinical Microbiological Reviews,* 8 (2) 161-179.
Retson, D. (1995), Coughing kids spur fungi find in school. *Medicine Hat News,* 16 January 1995.
Sternberg, S. (1994), The emerging fungal threat. *Science,* 266: 1632-1634.
Zuger, A. (1995), The terminator. *Discover,* January, 24-27.
Rogers, T.R., K.A. Haynes and R.A. Barnes. (1990), Value of antigen detection in predicting invasive pulmonary aspergillosis. *Lancet,* 336: 1210-1213.
Spreadbury, C., D. Holden, A. Aufauvre-Brown, B. Bainbridge and J. Cohen. (1993), Detection of *Aspergillus fumigatus* by polymerase chain reaction. *J. Clin. Microbiol.*, 31: 615-621.
Colwell, F.S. (1989), Microbiological comparison of surface soil and unsaturated subsurface soil from a semi-arid high desert. *Appl. Environ. Microbiol.*, 55: 1214-1219.

### Summary of the Invention

The present invention is directed to utilizing supernatants of yeast or fungi cells as serodiagnostic materials for detecting antibodies from subjects infected with the corresponding antigens.

More specifically, the present invention focuses on those military subjects who were engaged in the Persian Gulf Crisis in 1990-91. These subjects were chosen since most other possible causes had been eliminated, there was deep concern on determining the cause of their ailments and fungi as a possible cause had never been investigated.

The present invention also discloses certain unique characteristics of yeast and fungal supernatants which enhance the accuracy and efficacy of the serodiagnostic method of the invention. It has been found that supernatants of some fungi display "generic" affinity in that such supernatants can be used to detect serum antibodies to a large panel of different fungal species. In other instances, supernatants of some fungi display "specific" affinity such that only serum antibodies of the same fungus are capable of being detected.

Furthermore, the present invention provides that antigenicity of cell supernatants of yeast or fungi diminishes when placed in temperature approaching the freezing point. At freezing point, antigenicity is lost.

In accordance to the present invention, there provides a serodiagnostic assay for fungal antibody which comprises preparing fungal cell culture supernatants; reacting said fungal cell culture supernatants with sera from a test subject; and determining the serum antibody level of said test subject.

### Detailed Description of the Preferred Embodiment

The present invention focuses on fungi and yeast present in soil samples from the Middle East. In addition, when packages sent to the Middle East were returned, these were heavily contaminated, coated with a layer of fungi on the outside of the parcels. It was a concern that fungi might be a health hazard to those military personnel who may have been either exposed to these contaminated packages or those stationed in the Persian Gulf where these were present.

Also, subsequent to the Gulf War conflict, it was disclosed that Iraq had researched fungi causing crop diseases and aflatoxins as part of its biological program (Taylor, 1996, Zalinskas, 1997). However, given the heat, humidity and poor sanitation of some of the areas of Iraq, Allied military personnel in the area were more at risk in being exposed to endemic fungal diseases or food contamination than any biological warfare (BW) weapon.

This investigation sought to determine if there was any indication that fungal infections played a role in the health of veterans who had served in the Persian Gulf by applying a novel serodiagnostic assay.

The present invention provides that serum from mice vaccinated with fungal cells did not bind to supernatants from yeast cells, and vice versa. However, a combination assay (fungal and yeast antigens on the same plate) renders the detection possible. In addition, mice vaccinated with purified aflatoxins had sera which reacted very poorly to purified aflatoxins placed onto microtitre plates but reacted very strongly with the "generic" fungal supernatant.

According to the present invention, it has been found that one fungus, *Cladosporium*, had supernatant which gave false positives (i.e. it gave positive reaction for any antibody, even that of negative control mouse serum) under alkaline conditions but not under other conditions.

It has been found that fungi and yeast grow best at room temperature (e.g. 20 °C) and aeration (shaken cultures) rather than higher temperatures (e.g. 37 °C, some did not grow at all) and poor aeration (cultures left static). Initially a mouse model was used to develop confidence levels on what constituted a positive or negative serum result to a variety of fungi, yeast and their toxins. Although mice were vaccinated with killed washed cells, these vaccinated animals responded far greater to fungal or yeast culture supernatants.

### Materials and Methods

### (a) Fungal isolations:

Soil samples were obtained near the Canadian camps at Canada Dry 1 (noted as Qatar), Canada Dry 2 (noted as Doha) and Bahrain. In 1991, canisters containing the soil samples were opened and inspected at Defence Research Establishment Suffield (DRES) under BioContainment Level 3 (BL-3) conditions to safeguard against the remote possibility that pathogens may be present. *Brucella* species, *Francisella tularensis, Yersina pestis* and *Bacillus anthracis* were not found in these samples (results unpublished). For fungi that were present in these samples, single colonies were picked with a sterile loop and transferred to Trypticase-Soy™ (TS) agar plates. For a given sample, where fungal colonies appeared identical (colonial morphology, rate of growth and pigmentation were used to assess similarities), a representative colony was chosen for sub-culture.

Contaminated package material from the Middle East (Bahrain) was also taken into the BL-3 facilities at DRES for further inspection. Representative areas that were the most visibly contaminated were cut into 1 cm x 2 cm strips with sterilized scissors and placed into tubes of 9 ml 1% sterile saline and incubated at 37°C with gentle shaking for 1 hour. The liquid was then diluted, plated and incubated as noted above.

As the taxonomy of the fungi were beyond the capabilities of the DRES, specimens were sent to the National Centre for Human Mycotic Diseases (NCHMD, Edmonton, Alberta).

Baker's yeast (*Saccharomyces cerevisiae*) was obtained from a local grocery store.

### (b) Fungal/yeast cell antigens:

As the fungi and yeast organisms were from outside the country, to ensure the safety of the public, after identification these cultures were destroyed by autoclaving. Canadian representative cultures (for 13 of the identified fungal or yeast species found in the soil samples and contaminated packaging from the Middle East) were acquired from NCHMD. The cultures were grown on potato dextrose medium slants. Scrapings were taken off these slants and used to inoculate sterile potato dextrose broth (Difco Laboratories - 500 ml broth in a 1 L flask). Cultures were grown either statically or were shaken (100 rpm) for 2 weeks at 20°C (using an environmental shaker-incubator which can cool cultures below room temp).

For the immunization of mice, cultures were centrifuged at 15,000 X *g*, 4 °C, for 30 minutes, and the cellular pellets were saved. Part of each pellet (about 2 gm) was suspended in 1% saline with 2% phenol (40 ml) and shaken mechanically overnight at room temperature. The pellets were washed 3 times in sterile 1% saline, and then 200 mg of each was transferred to tubes containing 10 ml of sterile saline. The samples were homogenized with a Virtis Handishear™ (Canberra Packard Canada, Calgary) using three 10 second pulses at maximum speed (within an operational Biosafety Cabinet to confine aerosols). A syringe was loaded with this suspension (if the needle became plugged with cellular material, the loaded syringe was discarded and this procedure was repeated until an inoculum of suitable suspension was prepared). Mice were given 0.1 ml each of this suspension (2 mg wet weight of cellular material) at each immunization.

In one set of experiments, *Ulocladium* was prepared as above except that the final suspension was either in sterile 1% saline or 0.05 M sodium carbonate buffer (pH 9.6). One ml of each was either sonicated (5 µm amplitude, 8 cycles of 15 second sonications followed with 45 second pauses, chilled in an ice-water bath), autoclaved (15 psi, 121°C, 5 min.), or placed in a boiling water bath for 5 min. Also, in duplicate, 3 ml of mycelial suspension was extracted with an equal part of phenol, heated for 30 minutes at 70°C with stirring, then the phenol/water layers were separated with centrifugation at 15,000 X *g*, 4 °C, 30 min. Each layer was treated with 5 volumes of methanol with 1% sodium acetate (w/v) then left at 4 °C for 3 days. The precipitates were collected by centrifugation. For one set of aqueous or phenol layers, the pellet was dissolved in 1 ml of 1% sterile, for another set the pellet was dissolved in 1 ml 0.05 M carbonate buffer.

### (c) Fungal/yeast supernatant antigens:

Culture supernatant antigens were prepared from the cultures shaken for 2 weeks at 20 °C. Cultures were centrifuged at 20,000 X *g*, 4 °C, for 1 hour and the supernatants were then aseptically collected. (Note: a few cultures had to be centrifuged several times, a few others did not require centrifugation but instead their supernatants were removed after the cells settled to the bottom of the flask when left undisturbed for a few hours.)

Comparisons were done with part of the supernatants prepared as above, and the other part passed through 0.45 µm disposable vacuum filter units. For some culture supernatants, plugging of the filter was a difficulty. This was remedied by first passing small volumes (50 ml) of the unfiltered supernatant through a Whatman #1 filter paper with the assistance of a vacuum pump, removing the filtrate, changing the filter paper and then repeating with other small volumes. Once the supernatant had been pre-filtered, it was then filtered through a 0.45 µm disposable vacuum filter unit. The supernatants were kept at 4°C until required. It was observed that some of the supernatants lost their antigenicity upon freezing. To test antigen sensitivity to enzymes, DNAse, RNAse or protease (SIGMA Chemical Co., St. Louis) were added to aliquots of culture supenatants (final concentration 10 µg/ml) which were then incubated for 3 hours at 37 °C before being used in the ELISA.

### (d) Aflatoxins:

Aflatoxins B1, B2, G1 and G2 were purchased from Sigma Chemical Co. (St. Louis, Missouri). One mg of each was suspended in 10 ml of 0.2 µm filter-sterilized 1% NaCl and 10% glycerol. These were frozen at -70 °C until required.

### (e) Animal testing:

Male CD1 mice were purchased from Charles River Canada Inc., St. Contance, Quebec. Sets of 6 mice were used for each antigen tested. Control mice were injected with 0.1 ml saline. Test mice were given killed fungal or yeast cellular material (2 mg/0.1 ml sterile saline/mouse) or aflatoxins (10µg/0.1 ml sterile saline/mouse) by intra-peritoneal injection on weeks 0, 1, 2 and 6. To acquire blood samples, mice were first pre-warmed with a heat lamp, confined in a DRES-mouse restrainer, and then bled through the tail vein with a 26 gauge needle. About 0.3-0.5 ml was collected from each mouse. The blood was allowed to clot overnight at 4°C, samples were centrifuged in an Eppendorf™ Microfuge (14,000 rpm, 2 min, room temperature), and the sera was removed. An equivalent volume (to the serum removed) of 0.22µm sterilized 0.86% phosphate-buffered saline (PBS, Sigma, St. Louis, MI), 10% glycerol (a cryo-protectant) was added to the blood, the tube was vortexed then microfuged, the resultant supernatant was added to the serum, removed and this was then stored at -70 °C until required.

### (f) Enzyme-Linked Immunosorbent Assay (ELISA):

Solutions used were as noted below:
Coating buffer: 1.59 g Na₂CO₃, 2.93 g NaHCO₃, 0.2 g sodium azide, pH 9.6, triply-distilled water to 1 L, or 1% sterile saline depending on the situation.
Wash buffer: one package of prepared phosphate buffered saline (120 mmol NaCl, 2.7 mmol. KCl, 10 mmol phosphate, pH 7.4, Sigma Diagnostics, St. Louis, Missouri), 1 g bovine serum albumin (BSA), 0.5 ml Tween 20, water to 1 L.
Blocking buffer: one package of phosphate buffered saline, 20 g BSA, 1 ml Tween 20, water to 1 L.
Indicator antibody: 1 part goat anti-mouse (anti- IgG plus IgM) or 1 part goat anti-human horseradish peroxidase conjugate (Caltag Lab., San Fransisco, CA) was added to 1000 parts wash buffer.
Substrate solution: 1 part ABTS peroxidase substrate plus 1 part peroxidase solution B (Kirdegaard and Perry Lab. Inc.. Gaithersburg, Maryland). Aliquots of 100 µl of antigen (either cell preparations, culture supernatants, aflatoxin suspensions or, as a control, uninoculated potato dextrose medium) were added to each well of MaxiSorp Nunc-Immuno Plate™ (Gibco BRL, Burlington, Ontario). Then 100 µl of either sterile 1% saline or double-strength carbonate coating buffer were added, lids were placed on top of the microtitre plates and these were incubated overnight at room temperature (18 hr, 22 °C). After this time, plates were washed 5 times with wash buffer (300 µl/well), blocking buffer (200 µl/well) was added and the plates were incubated 1 hours at 37°C. Plates were washed 5 times with wash buffer, then 20 µl of test serum plus 80 µl of wash buffer were added to the wells and the plates were incubated 1 hour at 37 °C. The plates were washed 5 times with wash buffer, then 100 µl of indicator antibody solution/well was added and the plates were incubated 1 hour at 37 °C. The plates were washed 5 times with wash buffer then 200 µl of substrate solution/well was added and the plates were incubated for colour development. As not all antigens were equivalent, incubation times varied (from a few minutes at room temperature to as much as 30 minutes at 37 °C). As a guide to when to stop incubation, the plates were scanned at 405 nm and the readings were recorded when the positive control mouse serum gave readings around 0.600 and the negative control mouse serum was less than 0.200. For assays using human sera, negative and positive control mouse sera were also used, then goat anti-mouse indicator antibody was used in wells that had mouse sera, goat anti-human indicator antibody was used in wells that had human sera.

### (g) Testing of human sera:

Two groups of controls were used from volunteer staff at DRES. Five were staff that had been in the Persian Gulf and were in good health, 7 were staff that had not been in the Persian Gulf. From the United States, 28 were military personnel who had been in the Persian Gulf and who had non-specific and unidentified illnesses over several years. Sera were collected and frozen at -70 °C until used (no cryo-protectant such as glycerol was used).

### Results and Discussions

### (a) Fungal isolation:

It was observed that fungi were present as a common microbial component from soil samples found in the Middle East during the Persian Gulf Crisis. Also during this time, when packages were returned from this region, it was observed that these were heavily contaminated with fungal growth on the outside surface, possibly a result of wetting of the parcels during shipment and the paper/cardboard exterior acting as a nutritional substrate for saprophytic fungi. Tables I and II present some of the fungi identified from these samples. Some fungi failed to grow upon sub-culture, possibly a result of either their special nutritional requirements or growth at 37 °C before it was realized that some prefered cooler temperatures (20 °C). It was evident that both the soil samples and the contaminated returned parcels had diverse fungal flora. Regarding the soil samples, bacterial and fungal .populations were low, usually less than 1000 isolates per gram of soil. Low microbial numbers are typical for sandy dry desert soils (Colwell, 1989).

Although saprophytic fungi seldom affect people, infections do occur when they are either immuno-compromised such as when affected by AIDS, cancer chemotherapy, diabetes, antibiotics are used over continued time or when overwhelmed with a high inoculum. It seemed reasonable to speculate that the stresses of war might also contribute to patient susceptibility to fungal or yeast infections.From the soil samples or contaminated packages from the Middle East, fungi could be isolated, notably *Alternia, Aspergilllus, Cladosporium, Fusarium* and *Penicillium,* that have been reported as important causes of illness.

### (b) Fungal/yeast growth:

In the present invention, the inventor developed a serodiagnostic assay to screen fungal-specific antibodies produced after exposure to a high antigen concentration. The initial step was to produce suitable antigens. To avoid any concerns by regulatory agencies on the use of fungal or yeast cultures from outside the country, these were destroyed and in their place indigenous representative cultures were acquired from the National Centre for Human Mycotic Diseases (NCHMD, Edmonton, Alberta). When these fungi were incubated at 30-37 °C, some (e.g. *Phoma*) failed to grow. Instead, a cooler temperature of 20 °C proved ideal for the growth of these fungi. This may to the fact that these fungi usually dwell in Canadian soils or debris that are usually cool.

Table III shows the different cell yield depending on whether the cultures were left static or shaken, both for 2 weeks. For the latter, the pellets sometimes contained material other than mycelia such as extensive mucoid material which greatly contributed to the weights. This material must be very hydroscopic as 500 ml of potato dextrose broth only has 12 grams of medium. These cultures also varied in the extent and colour of pigmentation (data not shown).

### (c) Fungal/yeast cell antigens:

To prepare killed fungi as a source of antigen, the fungal species were treated with 2% phenol overnight with constant mechanical shaking, then washed with 1% sterile saline. It was subsequently found that, although this treatment appeared to sterilize most fungi, some cultures, such as *Aspergillus* and *Chaetomium,* did not appear affected. Either these fungi were resistant or used dilute phenol as a carbon source. Potentially these fungi can be used in bio-remediation or the digestion of toxic carbon-based spills in cleanups.

As a result of washing and dilutions, the serological reactions of the mice, therefore, was likely to have been due only in response to fungal or yeast antigens and not to the components of the media that might have otherwise been carried over as contaminants. (In support of this, ELISA results with the appropriate controls, later on in this text, showed that the immune response was to the fungal or yeast antigens and not to the potato-dextrose medium.)

As no human positive sera (i.e. from patients infected with known fungal species) were available to develop a detection assay at the time of the initial studies, a mouse model was used. Sets of 6 male CD1 mice were immunized with 2 mg of fungal mycelia/0.1 ml sterile saline/mouse on weeks 0, 1, 2 and 6. By the end of the experiment, some mice had died in these sets. It is unknown whether this was a result of susceptibility to the fungal inoculation or to infections and trauma that resulted from male/male in-fighting. Curiously, although the health of some mice (e.g. those inoculated with *Aspergillus* or *Neosartoya* mycelia) rapidly declined during the course of these experiments, for other mice (e.g. those inoculated with *Paecilomyces* and *Penicillium*) their health appeared to improve (greater activity, excellent fur coat lustre) as compared to the uninoculated control mice. Possibly this observation reflects the presence of fungal toxins or antibiotics, respectively, which may have influenced the health of the animals.

### (d) The unobvious use of culture supernatants, rather than mycelia/cells, as antigen in an immunological assay:

To select the best method for antigen preparation, a single fungal species was chosen. *Ulocladium*, which was prone to clumping even after homogenization, was viewed as the worst case. It was viewed that what worked for this species was likely to work for the others (e.g. *Alternaria, Aspergillus, Biopolaris, Chaetomium, Cladosporium, Fusarium, Neosartorya, Paecilomyces, Penicillium, Phoma and Stachybotrys*). The same can be said about yeast (e.g. *Saccharomyces cerevisiae*).

Table IV shows that, even though mice were immunized with washed cells, fungal component(s) shed into the culture supernatant were the best antigen for anti-fungal antibody detection in the ELISA. This reagent is associated with the cell (since washed cells induced an immunological response in mice) but is also released into the surrounding liquid.

The above findings showed that antigenicity of culture supernatants rather than fungal mycelia as a source of antigen for a fungal ELISA and could act as a source of antigen for a fungal ELISA used to inspect the serology of vaccinated mice. Anti-fungal antibodies from mice (sera was used from immunized animals bled on week 6) were readily detected (see Table V).

Controls also showed that this assay was specific for anti-fungal antibodies. Wells in columns 1 and 2 of the microtitre plate had sterile potato-dextrose broth and pooled antisera from mice immunized with fungi or yeast did not show any significant affinity for this broth as an antigen. Wells in columns 3 and 4 had fungal supernatants and sera from unimmunized mice did not appear to have detectable levels of antibodies that bound to these fungal antigens. In contrast, for wells in columns 5-12 coated with fungal supernatants, immunized mice did have antibodies in their sera that bound to these antigens. Table V gives a numerical presentation of these results.

It was observed that there was some variablility in the results of the assay depending on what the antigens (culture supernatants) were diluted in before being applied to the microtitre plate. For some (e.g. supernatants from *Alternaria* and *Chaetomium* cultures), when these were diluted 1:1 in sterile 1% saline and applied to the microtitre plate, antibodies from some of the mice immunized with cellular antigens bound about 20-40% more than when these fungal supernatants were diluted 1:1 with 0.05 M carbonate buffer (pH 9.6). For other supernatant antigens (e.g., *Aspergillus, Neosartorya* and *Penicillium*) when these were diluted 1:1 with carbonate buffer, about 20-40% more antibodies bound to these than when 1% sterile saline was used. For the remaining fungal antigens there was no discernable difference (data not shown). Perhaps these differences reflect different populations of secreted antigens with different sensitivities to environmental conditions. Overall, there did not appear to be any definitive advantage for using carbonate buffer as the diluent and so either culture supernatants were used directly from the bottle or these supernatants were diluted in saline as required.

It was also observed that when the culture supernatant of *Cladosporium* was added as antigen to the wells, without dilution, it gave expected results with control and immunized mouse sera. However, when an equal volume of double-strength carbonate buffer was added, antibodies from any mouse serum (whether negative control sera or sera from mice immunized with any of the fungi or yeast) gave a non-specific high positive reading (data not shown). Either *Cladosporium* secretes a substance that binds to the plastic plates and, when treated with alkaline, binds antibodies (much like protein A of *Staphylococcus aureus* binds antibodies) or else the carbonate activates a peroxidase secreted by this fungus that binds to the plates and hydrolyzes the horseradish peroxidase reagents. In any case, for this reason, culture supernatants were used as antigen either straight from the bottle or with dilution in saline rather than carbonate buffer.

A curious observation from Table V is that negative control sera placed on fungal/yeast supernatant antigens gave lower ELISA readings than if positive immunized sera was placed on potato-dextrose (sterile and uninoculated). Either there is some component in the fungal/yeast supernatants that reduced non-specific binding, or trace amounts of fungal/yeast antigens were present in the potato-dextrose medium. Although these differences were noticeable, these are trivial as these controls still gave far lower ELISA readings than immunized sera placed on the respective fungal supernatant antigens.

Table V also shows that anti-fungal antibodies did have detectable affinity for most of the fungal supernatants (for *Stachybotrys*, it was subsequently found that sera taken from mice at wk 10 of the experiment, rather than week 6. did give a positive reaction). Possibly far more significant ELISA readings would have resulted if purified fungal components in the supernatants had been used either to immunize mice or to serve as antigens in the ELISA assay.

To avoid the presence of fungi/yeast in the laboratory and to prevent possible degradation of the antigens due to fungal/yeast contaminants of the culture supernatants, a comparison was done between unfiltered and filtered (i.e. supernatants passed through a 0.45 µm Millipore™ filter) culture supernatants. Only *Alternaria* supematants were affected by filtration, losing about 60% of its antigenicity following filtration (data not shown). It is possible that a great deal of the antigens in its supernatants were due to mycelial fragments that were removed by filtration. Due to the above observation, fungal/yeast supernatants used in subsequent studies were filtered before being stored at 4 °C, except for *Alternaria* supernatants which were left unfiltered. Aside from *Alternaria* supernatants, it was observed that filtration reduced the high positive readings of mouse immunized sera only slightly while reducing the low negative control sera readings by about half (data not shown).

### (e) Preliminary characterization of the fungal/yeast supernatant antigens:

To determine what the antigenic components were in the noted fungal/yeast supernatants, these were treated with DNAse (digests DNA), RNAse (digests RNA) or protease (digests proteins) in 0.25 M TRIS buffer at pH 7.2 before being used in the ELISA. Mouse sera used were from wk 10 of the immunization series (i.e. the highest titres available). The noted enzymatic digestions only had a minor effect on the antigens. Most samples gave results similar to that of the controls for the ELISA reading, a few had reductions of 10-20% in the ELISA reading but there was no consistent pattern to these (data not shown). It appeared that the antigens were either resistant to these enzymes or were components other than nucleic acids or proteins. The latter is more probable, given that these organisms, such as fungi, produce a large range of mycotoxins, aflatoxins, aflatoxicols and fungal lipids (Sigma Chemical Co., St. Louis, MI). In support of this, when fungal/yeast supernatants were freeze-dried, the material made to 10 mg/ml in sterile saline and then run on an SDS-PAGE, only 4 protein bands of 17,000 to 19,500 m.w. were observed both for these preparations and for uninoculated potato-dextrose broth (data not shown).

It was hoped that the fungal/yeast culture supernatant antigens could be freeze-dried and stored indefinitely or available as a powder for reconstitution. When aliquots of these supernatants were freeze-dried, the supernatants no longer worked as antigen for the immunized sera (i.e. only negative results, similar to the controls, were observed, data not shown). When the supernatants were simply frozen, again the supernatant antigens did not appear to be recognized by the antibodies in the sera of immunized animals. Freezing, it appears, either denatured the antigens or caused interactions to occur that blocked presentation of key epitopes. Fungal culture supematants, therefore, were kept at 4 °C rather than frozen, until required. Most of these antigens were stable over prolonged storage as determined by similar reaction regardless whether the filter-sterilized supematants were stored for 2 years at 4 °C or prepared from fresh fungal/yeast cultures (data not shown).

### (f) Response of groups of mice vaccinated with fungi or yeast cells:

The mice, vaccinated with fungal or yeast cells treated with 2% phenol and washed 3 times in sterile saline, were bled over a 10 week study, the sera from each group pooled and kept frozen until use, and then all sera pools were applied to microtitre plates coated with respective culture supernatants. The results are noted in Table VI. As can be seen, there was considerable variation of the response of mice to the different vaccinations. The responses were very weak (405nm reading of 0-0.100, average of wells with antiserum minus wells with control naive serum), intermediate (405nm reading of 0.100-0.300) or very strong (405 nm reading greater than 0.300).

### (g) Cross-reaction of fungal/yeast antigens:

Although Table V shows that the modified ELISA assay detected anti-fungal or anti-yeast antibodies, it only gives part of the applications of the modified ELISA. It was investigated if it was also specific for the different fungal species tested. Two aspects of specificity were investigated:
(1) If different fungal/yeast supernatants were applied to the microtitre plates, how would a serum (or rather sera pool to a given culture) respond to these? Would antibodies in anti-*Aspergillus* antiserum only bind to *Aspergillus* supernatant antigen on the modified ELISA or would these also bind to supernatant antigens from all cultures?
   In this test, single antisera are applied to wells coated with different culture antigens. (see Table VII).
(2) How useful was each culture antigen? Would only *anti-Aspergillus* antiserum bind to *Aspergillus* supernatant antigen on the modified ELISA, or would all the other antisera bind to this same antigen? (see Table VIII).
   The two trials are the reverse of one another. In the first, one serum is applied to several antigens; in the second, several sera are applied to one antigen.

From Tables VI, VII and VIII, there are striking findings. Mice gave very low antibody responses when vaccinated with *Ulocladium* (Table VI). However, if the antigen on the microtitre plates is the supernatant of *Bipolaris,* rather than *Ulocladium,* sensitivity is increased 7-fold (Table VII). Upon inspecting Table VIII, although some fungal supernatants appear "generic" in that when *Aspergillus* is the source material most anti-fungal antisera will bind to this on the microtitre plates, others are "specific" in that *Fusarium* supernatant will bind very little other than mouse anti*-Fusarium* antibodies. Baker's yeast supernatant binds very little other than Baker's yeast though this is more likely to be due to antigenic differences between yeast and fungi than it is to specificity.

As can be seen from Table VII, species of fungal or yeast cell culture supernatants which demonstrate "specific" antibody affinity include *Alternaria, Baker's Yeast, Chaetomium* and *Fusarium*. Likewise, Table VIII shows those species of fungal or yeast cell culture supernatants which demonstrate "generic" antibody affinity include *Aspergillus* and *Paecilomyces.*

For the remaining species, namely *Bipolaris, Neosatorya, Penicillium, Stachybotrys* and *Uliocladium*, it can be seen that these antigens detect not only respective antisera antibodies but also a few antisera raised against other fungi. Applications of existing technologies (e.g. cross-adsorbsion, affinity columns) are suggested for making either the antigens (i.e. culture supernatants) or antisera more specific.

### (h) Toxins (aflatoxins):

Mice were vaccinated with aflatoxins B1, B2, G1 or G2 (see Materials and Methods), bled on week 10, and their sera were tested for antibodies that bound to various antigens. Table IX shows the different effectiveness for anti-aflatoxin serum antibody detection.

As can be seen from the above, some culture supernatants, such as those from *Alternia, Aspergillus* and *Penicillium,* are far better than the use of purified aflatoxins (10 ug/0.1ml) for detecting anti-aflatoxin antibodies in mouse sera. It was not determined whether this was a result of higher aflatoxin concentrations in the culture supernatants or whether components in the culture supernatants assisted the binding of the aflatoxins present. Regardless of this, it can be seen from this and the previous text that some culture supernatants not only detect exposure to fungal/yeast cells but also their toxins.

### (i) Testing human sera, controls and sera from ill Persian Gulf veterans:

Using the mouse model for confidence levels, whereby ELISA 405nm reading suggest that 0-0.300 are weak reactions, 0.300-0.600 are intermediate reactions, and anything greater than 0.600 is a strong reaction, human sera was applied onto the fungal/yeast supernatants. Three sources of sera were tested:
□ Civilians (7 people, Canadian) that had not been to the Persian Gulf
□ Either civilians or soldiers (5 people, Canadian) that had been to the Persian Gulf and were in good health
□ Veterans (28 people, American) that had been to the Persian Gulf and were in poor health

Table X shows how this sera bound to the fungal/yeast supernatant antigens in the ELISA.

From the above data, it can be seen that for a few of the fungi (e.g. *Bipolaris* and *Ulocladium*) the people tested in general have intermediate levels of antibodies to antigens in their culture supenatants. This is likely to be due to the long lifespan of humans during which time exposure to either these or contaminated foods are likely to contribute to the positive serology of the general population. In contrast, mice with a short lifespan, controlled water and food supply, are less likely to be exposed to these naturally and so had far lower antibody levels as noted in the text. The only exception appeared to be for *Chaetomium*. For this species, the average reading for Persian Gulf veterans sera tested on its culture supernatant was nothing unusual. However, upon entering the results into a computer program for averaging, it was clear that, unlike for the other fungal/yeast supernatant data, for *Chaetomium* supernatants the sera was not one but two groups. Twenty of the 28 subjects had low affinity for the antigens, but 8 of the 28 (about 30%) had very high affinity for *Chaetomium* supernatant antigens. It is unclear if this is "cause or effect". The further determination of whether *Chaetomium,* or possibly a related fungus found in the Gulf, cause the illness, or did the illness lead to a susceptibility to fungal infections such as *Chaetomium* is beyond the scope of this investigation.

It is to be understood that the embodiments and variations shown and described herein are merely illustrative of the principles of this invention and that various modifications may be implemented by those skilled in the art without departing from the scope and spirit of the invention.

The features disclosed in the foregoing description and in the claims may, both separately and in any combination thereof, be material for realising the invention in diverse forms thereof.

**Table I:**

| **Fungi isolated from soil samples from the Middle East** | |
|---|---|
| Soil Source | Fungal Species Isolated |
| Qatar (Canada Dry 1) | *Acremonium, Alternia, Bipolaris, Penicillium,* dark fungus which failed to grow on sub-culture (the bacterium Streptomyces was also common) |
| Doha (Canada Dry 2) | *Alternaria, Aspergillus glaucus* group, *Aspergillus versicolor*, *Aspergillus fumigatus, Chaetomium, Penicillium, Phoma, Microascus, Ulocladium,* an unidentified non-sporulating fungus (the bacterium *Streptomyces* also) |
| Bahrain | *Alternaria, Cladosporium, Fusarium, Paecilomyces lilacinus, Penicillium* |

**Table II:**

| **Fungi isolated from contaminated shipping material returned from the Middle East (Bahrain)** | |
|---|---|
| Cardboard Sample Number | Fungal Species Isolated |
| 1 | *Alternaria* |
| 2 | *Cladosporium, Penicillium* |
| 3 | *Aspergillus terreus, Penicillium* |
| 4 | *Penicillium* |
| 5 | dark fungus that failed to grow on sub-culture |
| 6 | dark fungus that failed to grow on sub-culture |
| *7* | *Alternaria, Aspergillus terreus, Cladosporium, Penicillium,* fungal cultures that failed to grow on sub-culture |
| *8* | *Acremonium, Aspergillus flavus, Dreschlera, Penicillium, Trichoderma* |
| 9 | *Alternaria, Aspergillus ustus, Aspergillus versicolor, Chaetomium, Penicillium, Paecilomyces, Stachybotrys* |
| 10 | *Alternaria, Aspergillus ustus, Chaetomium, Neosartorya, Penicillium, Ulocladium* |
| 11 | *Aspergillus ustus. Chaetomium, Penicillium* |

**Table III:**

| **Cell yield of different fungal species grown at 20 °C** | | |
|---|---|---|
| Fungal Species | Cell yield (grams wet weight for 500 ml culture) | |
| | Static Culture | Shaken Culture (100 rpm) |
| *Alternaria* | 4.9 | 42.1 |
| *Aspergillus* | 5.8 | 45.4 |
| Baker's Yeast | 1.6 | 6.8 |
| *Bipolaris*¹ | 5.6 | 135.7 |
| *Chaetomium* | 3.5 | 21.2 |
| *Cladosporium* | 1.6 | 30.1 |
| *Fusarium*¹ | 20.6 | 45.6 |
| *Neosartorya*¹ | 8.3 | 50.0 |
| *Paecilomyces* | 5.8 | 25.6 |
| *Penicillium* | 8.2 | 33.4 |
| *Phoma*¹ | 13.3 | 34.6 |
| *Stachybotrys* | 1.1 | 8.7 |
| *Ulocladium* | 7.6 | 41.8 |

| | | |
|---|---|---|
| ¹ Cultures that produced extensive slime and whose supernatants, used in serological assays noted later in the text, required pre-filtration before 0.45 µm filtration. | | |

**Table IV:**

| **Detection of mouse anti-fungal antibodies using different antigen preparations of *Ulocladium*** | |
|---|---|
| Antigen ^{a} | ELISA Readings (A 405 nm)^{b} |
| Control, no antigen | 0.027 ± 0.008 |
| **supernatant of fungal suspension** | **0.160** ± **0.004** |
| sonicated mycelia | 0.030 ± 0.008 |
| boiled (5 min) mycelia | 0.106 ± 0.013 |
| autoclaved (5 min) mycelia | 0.036 ± 0.006 |
| phenol layer, phenol-water extract | 0.030 ± 0.30 |
| aqueous layer, phenol-water extract | 0 ± 0 |

| | |
|---|---|
| ^{a} Antigens were suspended in either saline or carbonate coating buffer. Results are shown only for the former as no positive readings occurred when carbonate was used. | |
| ^{b} ELISA readings for blank wells (no antigen nor antisera) were subtracted from readings for control and test wells (which had antisera applied). | |

**Table V:**

| **ELISA readings for anti-fungal/yeast antibodies binding to antigens from fungal/yeast culture supernatants** | | |
|---|---|---|
| Antigen (Fungal/Yeast Culture Supernatants) | ELISA readings (A 405 nm)^{a} | |
| | Controls | Test sera |
| | Negative control sera | Anti-fungal antisera |
| *Alternaria* | 0.033 ± 0.030 | **0.249** ± **0.020** |
| *Aspergillus* | 0.018 ± 0.014 | **0.321** ± **0.108** |
| Baker's Yeast | 0.015 ± 0.010 | **0.195** ± **0.021** |
| *Bipolaris* | 0.025 ± 0.011 | **0.152** ± **0.048** |
| *Chaetomium* | 0.021 ± 0.006 | **0.129** ± **0.005** |
| *Cladosporium* | 0.029 ± 0.012 | **0.097** ± **0.012** |
| *Fusarium* | 0.060 ± 0.013 | **0.428** ± **0.196** |
| *Neosartorya* | 0.040 ± 0.011 | **0.156** ± **0.050** |
| *Paecilomyces* | 0.020 ± 0.004 | **0.100** ± **0.014** |
| *Penicillium* | 0.041 ± 0.011 | **0.557** ± **0.160** |
| *Phoma*^{b} | 0.020 ± 0.006 | **0.062** ± **0.008** |
| *Stachybotrys* | 0.021 ± 0.013 | **0.059** ± **0.010** |
| *Ulocladium* | 0.033 ± 0.020 | **0.153** ± **0.011** |

| | | |
|---|---|---|
| ^{a} ELISA readings for blank wells (no antigen nor sera) were subtracted from control and test wells (which had sera applied). Test antisera, that gave values at least 3 SD greater than for controls, are highlighted. Another control, placing sera from immunized mice onto potato dextrose medium as antigen coating the wells gave readings about twice (data not shown) that of the negative unimmunized sera on fungal/yeast supernatants shown above. | | |
| ^{b} Due to the initial failure of *Phoma* to grow at the time of this test, its data was entered at a date later than the others. | | |

**Table VI:**

| **Response of mice vaccinated with fungi or yeast cells** | | | | | | | | |
|---|---|---|---|---|---|---|---|---|
| | Antigen | week 1 serum | week 2 serum | week 3 serum | week 4 serum | week 5 serum | week 6 serum | wk 10 serum |
| Weak reaction | *Phoma* | 0.075 | 0.020 | 0.013 | 0.068 | 0.071 | 0.050 | 0.068 |
| | *Cladosp*. | 0.025 | 0.132 | 0.087 | 0.123 | 0.118 | 0.090 | 0.101 |
| | *Uloclad*. | 0.040 | 0.104 | 0.092 | 0.136 | 0.147 | 0.067 | 0.172 |
| | *Stachyr*. | 0 | 0.051 | 0.054 | 0.112 | 0.066 | 0.045 | 0.266 |
| | *Paecolm*. | 0 | 0 | 0 | 0.044 | 0.339 | 0.157 | 0.267 |
| Intermediate | Baker's Yeast | 0.048 | 0.075 | 0.135 | 0.150 | 0.143 | 0.149 | 0.397 |
| | *Chaetom*. | 0.051 | 0.116 | 0.055 | 0.204 | 0.245 | 0.313 | 0.415 |
| | *Neosatyr*. | 0.219 | 0.261 | 0.290 | 0.374 | 0.357 | 0.427 | 0.498 |
| | *Alternia* | 0 | 0.073 | 0.105 | 0.084 | 0.294 | 0.347 | 0.537 |
| Strong reaction | *Bipolaris* | 0.169 | 0.303 | 0.306 | 0.307 | 0.400 | 0.548 | 0.738 |
| | *Fusarium* | 0 | 0.352 | 0.382 | 0.311 | 0.283 | 0.518 | 0.830 |
| | *Aspergil.* | 0.058 | 0.363 | 0.366 | 0.390 | 0.551 | 0.824 | 0.814 |
| | *Penicill*. | 0.309 | 0.496 | 0.696 | 0.694 | 0.798 | 0.988 | 1.022 |
| Notes: Mice were immunized with 1 mg of 2% phenol treated then saline washed cells on weeks 0, 1, 2 and 6. The above readings are those for immunized mouse sera reacting with respective supernatant antigens minus control unimmunized mouse sera on the same antigens. The above are ELISA readings (405nm) related to the green colouration given by horse-radish peroxidase bound to goat-anti-mouse antiserum. To reduce clutter of the above table, standard deviations are not noted, these were about 10-30% of the averages (for 3 wells) shown. | | | | | | | | |

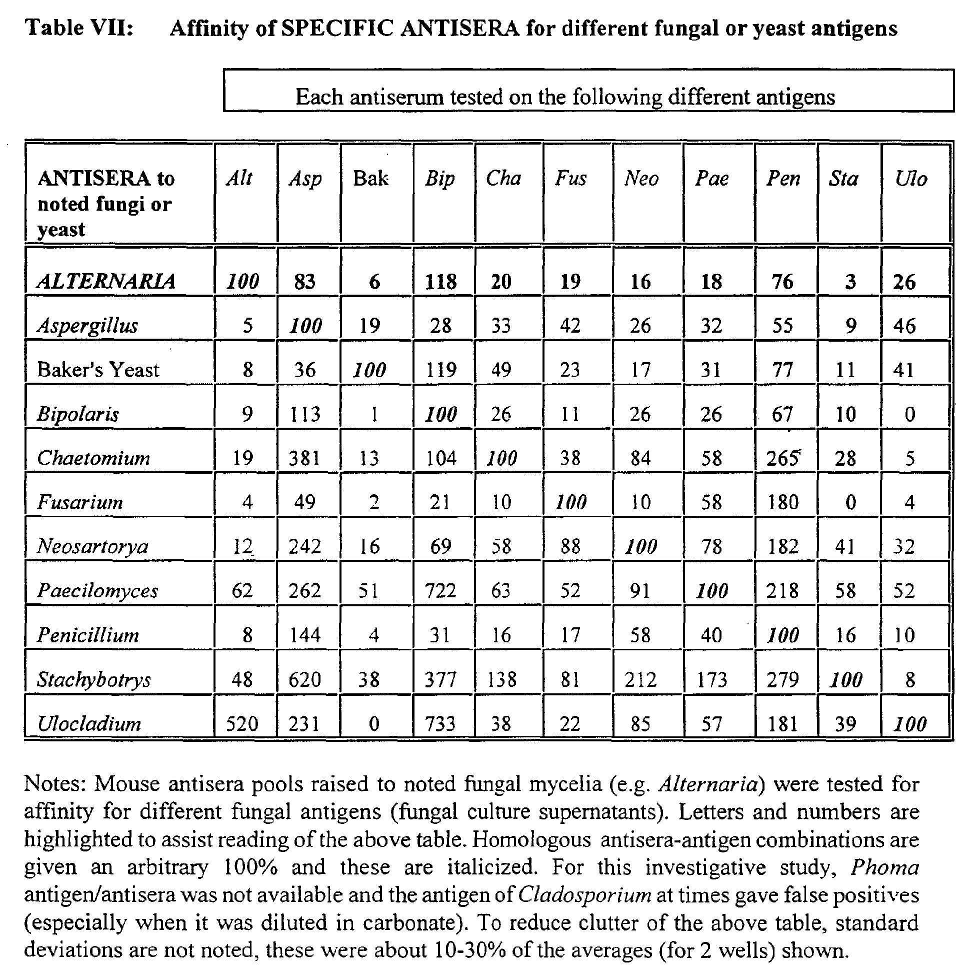

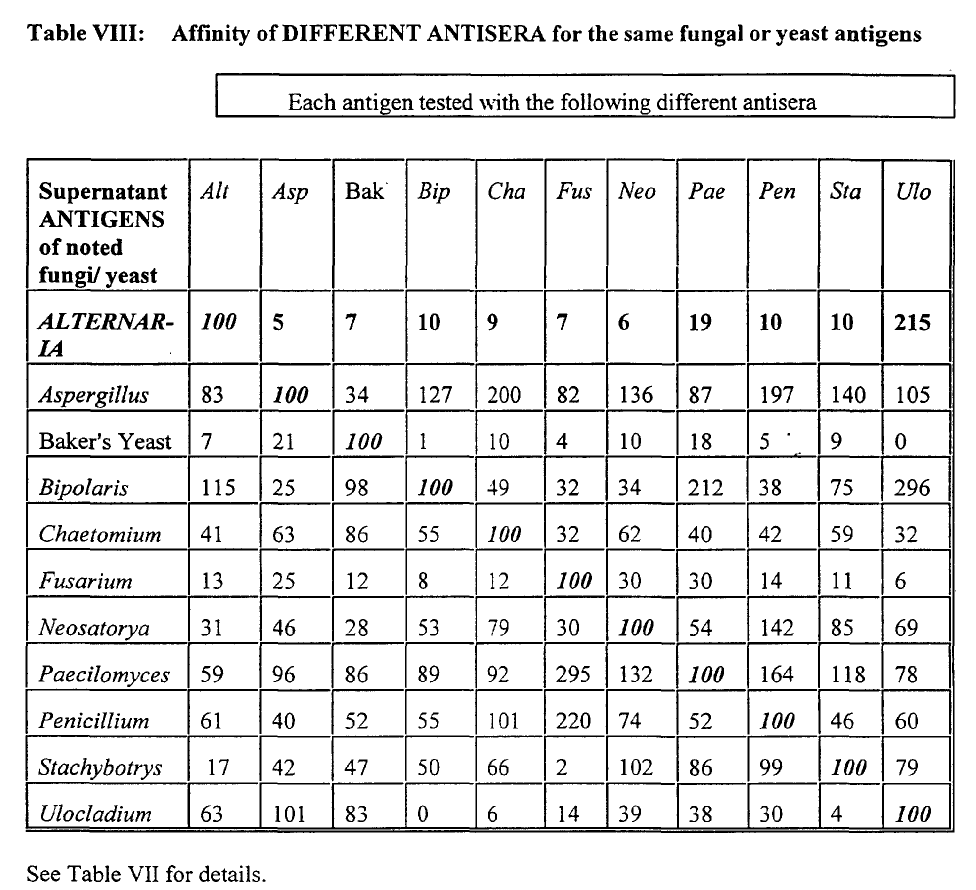

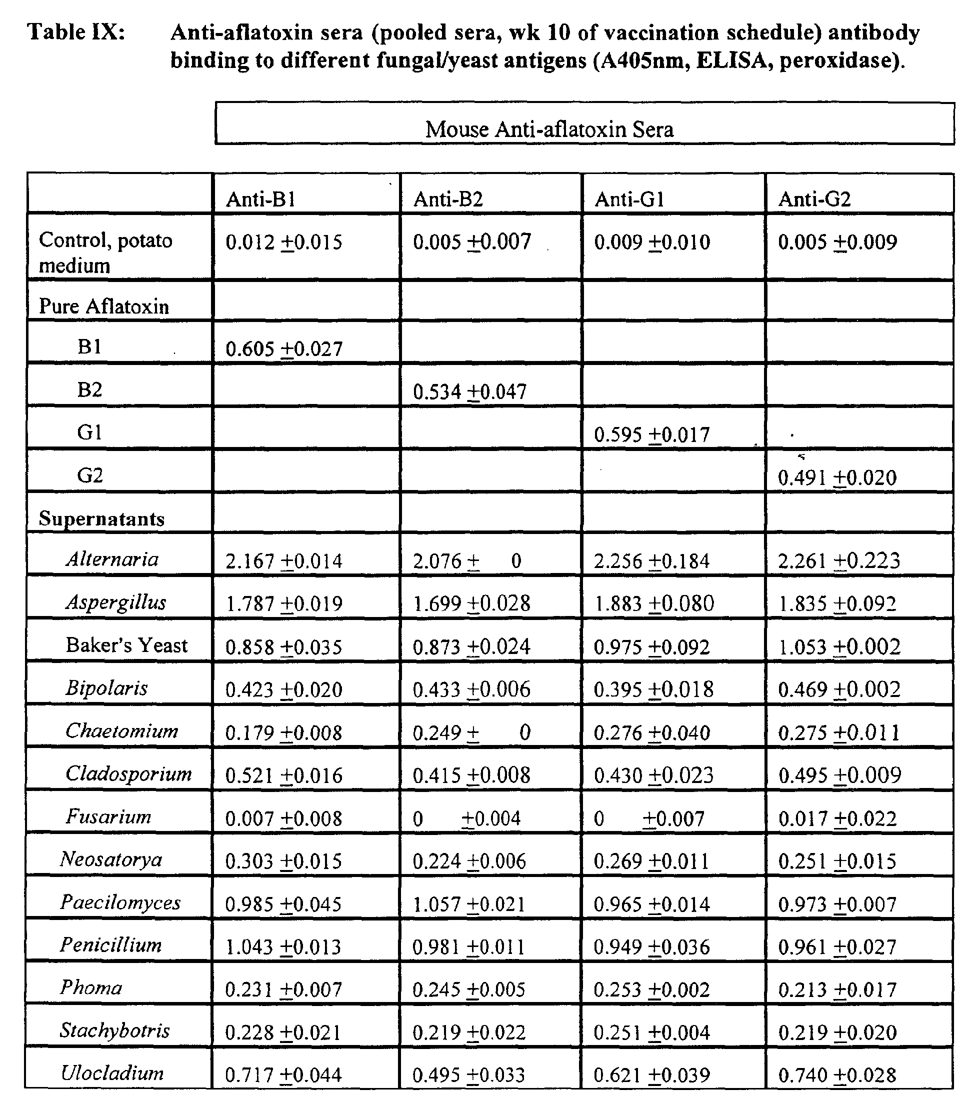

**Table X:**

| **ELISA readings for sera applied to microtitre plates coated with fungal/yeast culture supernatant antigens:** | | | |
|---|---|---|---|
| ANTIGEN culture supernatant | Sera, Controls Healthy Civilians (n=7) | Sera, Controls Healthy Veterans (n=5) | Sera, Test Samples, ill Persian Gulf veterans (n=28) |
| *Alternaria* | 0.166 ±0.052 | 0.228 ±0.063 | 0.149 ±0.062 |
| *Aspergillus* | 0.111 ±0.0.036 | 0.137 ±0.022 | 0.100 ±0.043 |
| Baker's Yeast | 0.118 ±0.0.70 | 0.171 ±0.124 | 0.115 ±0.080 |
| *Bipolaris* | 0.362 ±0.244 | 0.542 ±0.233 | 0.371 ±0.236 |
| *Chaetom.* | 0.076 ±0.166 | 0.011 ±0.049 | 0.316 ±0.311 (subgr=8) 0.737 ±0.391 (subgr=20)0.100±0.141 |
| *Cladospor*. | 0.180 ±0.119 | 0.174 ±0.043 | 0.172 ±0.125 |
| *Fusarium* | 0.311 ±0.109 | 0.303 ±0.041 | 0.238 ±0.083 |
| *Neosatyr.* | 0.174 ±0.041 | 0.173 ±0.033 | 0.077 ±0.037 |
| *Paecilomy*. | 0.184 ±0.047 | 0.154 ±0.023 | 0.168 ±0.089 |
| *Penicillium* | 0.213 ±0.057 | 0.172 ±0.055 | 0.148 ±0.129 |
| *Phoma* | 0.141 ±0.034 | 0.161 ±0.033 | 0.110 ±0.075 |
| *Stachybotr*. | 0.176 ±0.039 | 0.186 ±0.039 | 0.160 ±0.054 |
| *Ulocladium* | 0.300 ±0.041 | 0.318 ±0.050 | 0.241 ±0.057 |

## Claims

1. A serodiagnostic assay for fungal antibody comprising:
(i) preparing fungal cell culture supernatants;
(ii) reacting said fungal cell culture supernatants with sera from a test subject; and
(iii) determining the serum antibody level of said test subject.

2. The serodiagnostic assay of claim 1, wherein said fungal cell culture supernatants in steps (i) and (ii) are prepared and used at a temperature above the freezing point.

3. The serodiagnostic assay of claims 1 and 2, wherein said fungal cell culture supernatants in steps (i) and (ii) are prepared and used at 20 °C.

4. The serodiagnostic assay of claims 1 to 3, wherein said fungal cell culture supernatants in steps (i) and (ii) are prepared under aeration condition.

5. The serodiagnostic assay of claim 4, wherein said aeration condition is provided by gentle shaking.

6. The serodiagnostic assay of claim 1, wherein enzyme-linked immunosorbent assay (ELISA) is used in step (iii) for determining the serum antibody level.

7. The serodiagnostic assay of claims 1 to 6, wherein said assay is for the detection of yeast antibodies and said fungal cell culture supernatants are substituted with yeast cell culture supernatants.

8. A serodiagnostic assay for fungal and yeast antibodies comprising:
(i) preparing fungal and yeast cell culture supernatants mixture;
(ii) reacting said fungal and yeast cell culture supernatants with sera from a test subject; and
(iii) determining the serum antibody level of said test subject.

9. The serodiagnostic assay according to claims 1 to 8 wherein the test subject is animal or human.

10. A fungal or yeast cell culture supernatant as antigenic source for detecting level of antibodies from a sample test subject.

11. The fungal or yeast cell culture supernatant of claim 10, wherein said supernatant is prepared and used at a temperature above the freezing point.

12. The fungal or yeast cell culture supernatant of claim 11, wherein said supernatant is prepared and used at 20 °C.

13. The fungal or yeast cell culture supernatant of claim 11, wherein said supernatant is prepared under aeration condition.

14. The fungal or yeast cell culture supernatant of claim 13, wherein said aeration condition is provided by gentle shaking.

15. The fungal or yeast cell culture supernatant of claims 10 to 14, wherein said supernatant displays specific antibody affinity such that only serum antibodies of the same fungus or yeast are capable of being detected.

16. The fungal or yeast cell culture supernatant of claim 15, consisting of species selected from the group of *Alternaria, Baker's Yeast, Chaetomium* and *Fusarium*.

17. The fungal or yeast cell culture supernatant of claims 10 to 14, wherein said supernatant displays generic antibody affinity such that said supernatant can be used to detect serum antibodies to a large panel of different fungal or yeast species.

18. The fungal or yeast cell culture supernatant of claim 17, consisting of species selected from *Aspergillus* and *Paecilomyces.*

19. The fungal or yeast cell culture supernatant of claims 10 to 14, wherein said supernatant displays antibody affinity towards serum antibodies of the same fungal or yeast species as well as serum antibodies of other fungal or yeast species.

20. The fungal or yeast cell culture supernatant of claim 19, consisting of species selected from the group of *Bipolaris, Neosatorya, Penicillium, Stachybotrys* and *Uliocladium.*

21. The fungal or yeast cell culture supernatant of claims 10 to 14 displaying varying degrees of antigenicity.

22. The fungal or yeast cell culture supernatant of claims 10 to 14 effective in detecting aflatoxins.

23. The fungal cell culture supernatant of *Bipolaris* displaying significant degree of antigenicity towards antibody detection in the assay according to claim 1.

24. The fungal cell culture supernatant of *Cladosporium* displaying false positive indication under alkaline condition towards antibody detection in the assay according to claim 1.

25. The use of fungal or yeast cell culture supernatant as vaccines.

26. A fungal or yeast cell culture supernatant comprising only partially of nucleic acids or proteins.
